(19) Europäisches Patentamt
European Patent Office
Office européen des brevets

(11) Veröffentlichungsnummer: **0 021 327**
**A1**

(12) **EUROPÄISCHE PATENTANMELDUNG**

(21) Anmeldenummer: **80103374.7**

(22) Anmeldetag: **18.06.80**

(51) Int. Cl.³: **C 07 D 249/08**, A 01 N 43/64

(30) Priorität: **28.06.79 DE 2926096**

(43) Veröffentlichungstag der Anmeldung: **07.01.81 Patentblatt 81/1**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Zeeh, Bernd, Dr., Thorwaldsenstrasse 5, D-6700 Ludwigshafen (DE)**
Erfinder: **Ammermann, Eberhard, Dr., Sachsenstrasse 3, D-6700 Ludwigshafen (DE)**
Erfinder: **Sauter, Hubert, Dr., Neckarpromenade, D-6800 Mannheim 1 (DE)**
Erfinder: **Pommer, Ernst-Heinrich, Dr., Berliner Platz 7, D-6703 Limburgerhof (DE)**

(54) **Neue beta-Triazolylether, ihre Herstellung, sie enthaltende Fungizide, Verfahren zur Bekämpfung von Fungi und zur Herstellung von Fungiziden.**

(57) Die vorliegende Erfindung betrifft neue wertvolle β-Triazolylether mit guter fungizider Wirkung, Fungizide, die diese Verbindungen als Wirkstoffe enthalten und Verfahren zur Bekämpfung von Pilzen mit diesen Verbindungen.

Die β-Triazolylether besitzen die Formel

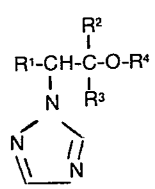

I,

in welcher

$R^1$ $C_{1-6}$-Alkyl oder gegebenenfalls halogensubstituiertes Benzyl,

$R^2$ $C_{1-6}$-Alkyl,

$R^3$ Wasserstoff, $C_{1-6}$-Alkyl, $C_{2-6}$-Alkenyl, $C_{2-6}$-Alkinyl oder gegebenenfalls halogensubstituiertes Benzyl und

$R^4$ einen $C_{1-6}$-Alkyl-, $C_{3-6}$-Alkenyl-, $C_{3-6}$-Alkinyl-, oder einen gegebenenfalls halogensubstituierten Benzylrest,

bedeuten.

EP 0 021 327 A1

Neue beta-Triazolylether, ihre Herstellung, sie enthaltende Fungizide, Verfahren zur Bekämpfung von Fungi und zur Herstellung von Fungiziden

Die vorliegende Erfindung betrifft neue ß-Triazolylether, Verfahren zu deren Herstellung, deren Verwendung als Fungizide, fungizide Mischungen, die diese Wirkstoffe enthalten, Verfahren zur Herstellung solcher fungizider Mischungen sowie Verfahren zur Bekämpfung von Schadpilzen mit diesen Fungiziden oder mit fungiziden Mischungen, die diese Verbindungen enthalten.

Es ist bekannt, daß ß-Imidazolylether wie beispielsweise das 1-(2'-(2",4"-Dichlorphenyl)-2'-(2"-propenyloxy)-ethyl)--1H-imidazol (DE-OS 20 63 857) fungizid wirksam ist. Dessen Wirkung, insbesondere gegen Mehltau- und Rostpilze in Getreide, ist jedoch unzureichend. Ferner sind ß-Triazolylalkohole aus der DE-OS 27 37 489 bekannt. Aber auch deren Wirkung, insbesondere gegen Mehltau- und Rostpilze in Getreide, ist insbesondere bei geringen Aufwandmengen unbefriedigend. Daher sind sie zur Bekämpfung von Schadpilzen im Pflanzen- und Materialschutz wenig geeignet.

Es wurde nun gefunden, daß die neuen ß-Triazolylether der Formel I

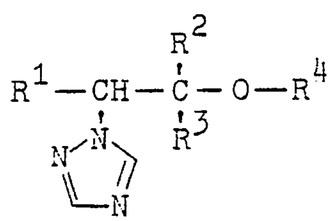

I,

WK/BL

in welcher

$R^1$ $C_{1-6}$-Alkyl oder gegebenenfalls halogensubstituiertes Benzyl,

$R^2$ $C_{1-6}$-Alkyl

$R^3$ Wasserstoff, $C_{1-6}$-Alkyl, $C_{2-6}$-Alkenyl, $C_{2-6}$-Alkinyl oder gegebenenfalls halogensubstituiertes Benzyl und

$R^4$ einen $C_{1-6}$-Alkyl-, $C_{3-6}$-Alkenyl-, $C_{3-6}$-Alkinyl oder einen gegebenenfalls halogensubstituierten Benzylrest

bedeuten, sowie deren für Pflanzen verträgliche Salze und Metallkomplexe eine sehr gute fungizide Wirksamkeit besitzen.

In der Formel I steht $R^1$ vorzugsweise für einen Alkylrest mit 1 bis 5 Kohlenstoffatomen, der unverzweigt sein kann wie beispielsweise ein Methyl-, Ethyl-, n-Propyl-, n-Butyl- oder n-Pentylrest oder verzweigt sein kann wie beispielsweise ein Isopropyl- oder ein Isobutylrest. Ferner steht $R^1$ für einen unsubstituierten oder durch Halogen, vorzugsweise Chlor, mono- oder disubstituierten Benzylrest.

$R^2$ steht vorzugsweise für einen Alkylrest mit 1 bis 4 Kohlenstoffatomen, der unverzweigt sein kann wie beispielsweise ein Methyl-, Ethyl-, n-Propyl- oder ein n-Butylrest oder verzweigt sein kann wie beispielsweise ein Isopropyl- oder ein tert.-Butylrest.

$R^3$ steht vorzugsweise für Wasserstoff oder für einen unverzweigten Alkylrest mit 1 bis 6 Kohlenstoffatomen wie beispielsweise für einen Methyl-, Ethyl-, Propyl-, Butyl- oder Pentylrest, für einen verzweigten Alkylrest mit 3 bis 6 Kohlenstoffatomen wie für einen Isopropyl- oder iso-Butylrest, für einen Alkenyl- oder Alkinylrest mit 2 bis

6 Kohlenstoffatomen wie für einen Vinyl-, Ethinyl-, Propen-2-yl-1-, Propin-2-yl-1- oder für einen 3-Methylbuten-2-yl-1-rest oder für einen gegebenenfalls durch Halogen, vorzugsweise Chlor, mono- oder disubstituierten Benzylrest.

$R^4$ steht vorzugsweise für einen Methyl-, Ethyl-, n-Propyl- oder n-Butyl-, Isopropyl-, ein 2-Methylpropyl-1- oder tert.-Butyl-, Propen-2-yl-1-, Propin-2-yl-1-, Cyclopentyl-, Cyclohexen-2-yl-1- oder Cyclohexylrest, ferner für einen gegebenenfalls durch Fluor oder Chlor, mono- oder disubstituierten Benzylrest.

Die neuen ß-Triazolylether besitzen im C-Atom, an das das Triazol gebunden ist, und - falls $R^2$ und $R^3$ unterschiedlich sind - im Carbinol-C-atom zwei Asymmetriezentren. Je nach der Beschaffenheit von $R^4$ kommen eventuell weitere Asymmetriezentren dazu. Mit üblichen Trennmethoden können die Verbindungen in Form einheitlicher Diastereomere erhalten werden. In der Praxis kann man sowohl die einheitlichen Diastereomeren wie auch die bei der Synthese üblicherweise anfallenden Gemische verwenden.

Die ß-Triazolylether der Formel I lassen sich herstellen, indem man Alkohole der Formel II

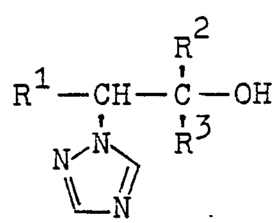

II,

worin $R^1$, $R^2$ und $R^3$ die oben angegebenen Bedeutungen haben, oder ihre Alkali- oder quartären Ammoniumsalze mit

einem Alkylierungsmittel der Formel III

$$L - R^4 \qquad III,$$

worin $R^4$ die oben angegebene Bedeutung hat und L für eine nucleophil verdrängbare Abgangsgruppe steht, gegebenenfalls in Gegenwart eines Lösungs- oder Verdünnungsmittels gegebenenfalls in Gegenwart anorganischer oder organischer Basen und/oder eines Reaktionsbeschleunigers bei Temperaturen zwischen 0 und 100°C umsetzt.

Für die im obigen Verfahren erwähnte nucleophil verdrängbare Abgangsgruppe L seien beispielsweise genannt: Halogen, vorzugsweise Chlor, Brom oder Iod; Alkylsulfat, vorzugsweise Methylsulfat; gegebenenfalls substituierte Alkylsulfonyloxyreste, vorzugsweise Methansulfonyloxy- oder Trifluormethansulfonyloxyreste; oder Arylsulfonyloxyreste, vorzugsweise der Tosylatrest.

Geeignete anorganische oder organische Basen, die gegebenenfalls auch als säurebindende Mittel in die Reaktion eingesetzt werden können, sind beispielsweise Alkali- und Erdalkalihydroxide wie Natriumhydroxid, Kaliumhydroxid, Calciumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dimethylcyclohexylamin, N-Methylpiperidin oder Pyridin. Es können aber auch andere übliche Basen verwendet werden.

Mit geeigneten Basen wie z.B. Alkalihydrid wie Natriumhydrid oder Lithiumalkylen wie Butyllithium oder mit Alkali- oder Erdalkalialkoholaten wie Natriummethylat können die Alkohole der Formel II auch in einer vorgeschalteten Umsetzung zunächst in ihre Alkoholat-Salze überführt werden und dann als solche zur Reaktion gebracht werden.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petrolether, Benzol, Toluol oder Xylole, Ester wie Essigsäureethylester, Amide wie Dimethylformamid, Nitrile wie Acetonitril, Sulfoxide wie Dimethylsulfoxid, Ketone wie Aceton oder Methylethylketon, Ether wie Diethylether, Tetrahydrofuran oder Dioxan oder entsprechende Gemische.

Als Reaktionsbeschleuniger kommen vorzugsweise Metallhalogenide wie Kaliumiodid, Kronenether, quartäre Ammoniumverbindungen wie Tetrabutylammoniumiodid oder Säuren oder Kombinationen dieser Reaktionsbeschleuniger in Frage.

Die erfindungsgemäßen Umsetzungen werden im allgemeinen bei Temperaturen zwischen 0 und 150°C in einem Zeitraum von 1 bis 60 Stunden durchgeführt, drucklos oder unter Druck, kontinuierlich oder diskontinuierlich.

Zur Isolierung der Verbindungen der Formel I wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden.

Falls gewünscht, können die ß-Triazolylether der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die ß-Triazolylether der Formel I nach bekannten Methoden in Metallkomplexe überführen. Das kann durch Umsetzung dieser Ether mit geeigneten Metallsalzen wie beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die zur Synthese der erfindungsgemäßen ß-Triazolylether eingesetzten Alkohole der Formel II

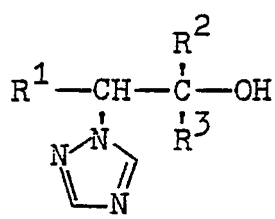

II,

worin $R^1$, $R^2$ und $R^3$ die oben erläuterten Bedeutungen haben, sind teilweise bekannt (DE-OS 27 37 489) oder können nach bekannten Methoden aus geeigneten α-Triazolylketonen hergestellt werden, beispielsweise durch Reduktion der Ketone mit komplexen Hydriden oder durch Umsetzung der Ketone mit Grignard-Reagentien.

Die Herstellung der erfindungsgemäßen Verbindungen der Formel I wird durch folgende Beispiele erläutert:

Beispiel 1

Zu einer Suspension von 1,6 g Natriumhydrid in 80 ml Tetrahydrofuran wird unter Stickstoffatmosphäre eine Lösung von 16,4 g 1-(2,4-Dichlorphenyl)-2-(1,2,4-triazol-1-yl)-4,4-dimethylpentanol-3 in 70 ml Tetrahydrofuran getropft und anschließend wird das Reaktionsgemisch für 2 Stunden bei Rückflußtemperatur nachgerührt. Dann werden bei Raumtemperatur 5,2 ml Allylbromid zugetropft. Nach Rühren über Nacht wird das überschüssige Natriumhydrid mit 150 ml Wasser zersetzt. Die wäßrige Phase wird dreimal mit je 100 ml Diethylether extrahiert, die vereinigten organischen Phasen werden dreimal mit 50 ml Wasser gewaschen, getrocknet und eingeengt.

Es verbleiben 16,5 g kristalliner 1-(2,4-Dichlorphenyl)-2-(1,2,4-triazol-1-yl)-4,4-dimethylpentyl-3-allylether vom Schmelzpunkt 90 bis 95°C.

| | | | | |
|---|---|---|---|---|
| ber.: | C 58,7 | H 6,3 | N 11,4 | Cl 19,3 |
| gef.: | C 58,6 | H 6,3 | N 11,3 | Cl 19,2 |

Beispiel 2

Zu einer Suspension von 1,6 g Natriumhydrid in 100 ml Tetrahydrofuran wird eine Lösung von 15,4 g 1-(4-Chlorphenyl)-2-(1,2,4-triazol-1-yl)-3,4,4-trimethylpentanol-3 in 100 ml Tetrahydrofuran getropft. Anschließend wird das Gemisch 2 Stunden lang bei der Rückflußtemperatur gerührt. Nach dem Abkühlen auf Raumtemperatur werden 3,7 ml Methyliodid zugegeben, wobei die Temperatur auf 30°C ansteigt. Nach fünfstündigem Rühren bei Rückflußtemperatur werden 200 ml Wasser zugegeben. Das Reaktionsgemisch wird dreimal mit je 100 ml Diethylether extrahiert, die vereinigten Etherphasen werden dreimal mit je 50 ml Wasser gewaschen,

getrocknet und eingeengt. Aus Petrolether kristallisieren 9,5 g 1-(4-Chlorphenyl)-2-(1,2,4-triazol-1-yl)-3,4,4-trimethylpentyl-3-methylether vom Schmelzpunkt 125 bis 127°C.

| | | | |
|---|---|---|---|
| ber.: | C 63,4 | H 7,5 | N 13,1 |
| gef.: | C 62,2 | H 7,4 | N 12,8 |

Analog Beispiel 1 und 2 wurden folgende Verbindungen der Formel I hergestellt:

| Bsp. Nr. | $R^1$ | $R^2$ | $R^3$ | $R^4$ | Fp (°C) |
|---|---|---|---|---|---|
| 3 | $4-Cl-C_6H_4-CH_2$ | $tert-C_4H_9$ | H | $C_2H_5$ | 106-108 |
| 4 | $4-Cl-C_6H_4-CH_2$ | $tert-C_4H_9$ | H | $CH_2-CH=CH_2$ | 105-107 |
| 5 | $4-Cl-C_6H_4-CH_2$ | $tert-C_4H_9$ | H | $CH_2-C\equiv CH$ | 98-100 |
| 6 | $4-Cl-C_6H_4-CH_2$ | $tert-C_4H_9$ | H | $CH_2-4-Cl-C_6H_4$ | 75- 77 |
| 7 | $2,4-Cl_2-C_6H_3-CH_2$ | $tert-C_4H_9$ | H | $C_2H_5$ | 102-104 |
| 8 | $2,4-Cl_2-C_6H_3-CH_2$ | $tert-C_4H_9$ | H | $CH_2-C_6H_5$ | 96- 98 |
| 9 | $2,4-Cl_2-C_6H_3-CH_2$ | $tert-C_4H_9$ | H | $CH_2-4-Cl-C_6H_4$ | 125-127 |
| 10 | $2,4-Cl_2-C_6H_3-CH_2$ | $tert-C_4H_9$ | $CH=CH_2$ | $CH_3$ | |
| 11 | $2,4-Cl_2-C_6H_3-CH_2$ | $tert-C_4H_9$ | $CH_3$ | $CH_2-CH=CH_2$ | |
| 12 | $2,4-Cl_2-C_6H_3-CH_2$ | $tert-C_4H_9$ | $CH=CH_2$ | $C_2H_5$ | |
| 13 | $2,4-Cl_2-C_6H_3-CH_2$ | $tert-C_4H_9$ | $CH_3$ | $CH_2-4-Cl-C_6H_4$ | |
| 14 | $2,4-Cl_2-C_6H_3-CH_2$ | $tert-C_4H_9$ | $CH=CH_2$ | $CH_2-C_6H_5$ | |
| 15 | $n-C_3H_7$ | $C_2H_5$ | H | $CH_3$ | |
| 16 | $n-C_3H_7$ | $C_2H_5$ | H | $CH_2-CH=CH_2$ | |
| 17 | $n-C_3H_7$ | $C_2H_5$ | H | $CH_2-C_6H_5$ | $n_D^{20}$ = 1.5130 |
| 18 | $n-C_3H_7$ | $C_2H_5$ | H | $CH_2-2,4-Cl_2-C_6H_3$ | Öl |
| 19 | $n-C_3H_7$ | $C_2H_5$ | $CH_3$ | $C_2H_5$ | |
| 20 | $C_6H_5-CH_2$ | $tert-C_4H_9$ | H | $C_2H_5$ | 79-81 |
| 21 | $4-Cl-C_6H_4-CH_2$ | $tert-C_4H_9$ | H | $CH_2-C_6H_5$ | 73-75 |
| 22 | $C_6H_5-CH_2$ | $tert-C_4H_9$ | H | $CH_2-CH=CH_2$ | 64-65 |
| 23 | $C_6H_5-CH_2$ | $tert-C_4H_9$ | H | $CH_3$ | |
| 24 | $C_6H_5-CH_2$ | $tert-C_4H_9$ | H | $2,4-Cl_2-C_6H_3-CH_2$ | |
| 25 | $4-Cl-C_6H_4-CH_2$ | $tert-C_4H_9$ | $CH_3$ | $CH_2-CH=CH_2$ | Öl |
| 26 | $2,4-Cl_2-C_6H_3-CH_2$ | $tert-C_4H_9$ | H | $CH_2-C\equiv CH$ | 70-72 |

Die erfindungsgemäßen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten:
Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Rhynchosporium secale an Getreide,
Venturia inaequalis (Apfelschorf),
Botrytis cinerea an Erdbeeren und Reben.

Die Verbindungen werden angewendet, indem man die Pflanzen mit den Wirkstoffen besprüht oder bestäubt oder die Samen der Pflanzen mit den Wirkstoffen behandelt. Die Anwendung

erfolgt vor oder nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wassser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch in Materialschutz u.a. zur

Bekämpfung holzzerstörender Pilze wie Coniophora puteanea und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 % (Gew.%) Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.% des Wirkstoffs enthält.

IV. 20 Gewichtsteile der Verbindung des Beispiels 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.% des Wirkstoffs enthält.

V. 20 Gewichtsteile der Verbindung des Beispiels 7 werden den mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20.000 Gewichtsteilen Wasser erhält man eine Spritzbrühe, die 0,1 Gew.% des Wirkstoffs enthält.

VI. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100.000 Gewichtsteilen Wasser erhält man eine wäßrige Dispersion, die 0,04 Gew.% Wirkstoff enthält.

IX. 20 Teile der Verbindung des Beispiels 4 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält

BAD ORIGINAL

man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Substanzen, wie
N-(1,1,2,2-Tetrachlorethylthio)-tetrahydrophthalimid,
N-Trichlormethylthio-tetrahydrophthalimid,

N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und weiter Substanzen wie
Dodecylguanidinacetat,
3-(3-(3,5-dimethyl-2-oxycyclohexyl)-2-hydroxyethyl)-glutaramid,
Hexachlorbenzol,
N-Dichlorfluormethylthio-N',N'-dimethyl-N-phenyl-schwefelsäurediamid,

2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolyl-harnstoff.

Die folgenden Beispiele A und B zeigen die biologische Wirkung der neuen Substanzen. Als Vergleichssubstanz diente das aus der DE-OS 20 63 857 bekannte 1-(2'-(2",4"-Dichlorphenyl)-2'-(2"-propenyloxy)-ethyl-1H-imidazol.

Beispiel A

Blätter von in Töpfen gewachsenen Weizenkeimlingen der Sorte "Jubilar" werden mit wäßrigen Emulsionen aus 80 % (Gew.%) Wirkstoff und 20 % Emulgiermittel besprüht und

nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt.

| Wirkstoff des Beispiels | Befall der Blätter nach Spritzung mit ...%-iger Wirkstoffbrühe | |
|---|---|---|
| | 0,012 | 0,006 |
| 1 | 0 | 0 |
| 3 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 18 | 0 | 1 |
| 20 | 0 | 1 |
| 21 | 0 | 0 |
| 22 | 0 | 1 |
| 25 | 0 | 2 |
| Vergleichssubstanz | 3 | 4 |
| Kontrolle (unbehandelt) | 5 | |

0 = kein Pilzbefall, abgestuft bis 5 = Totalbefall

Beispiel B

Blätter von in Töpfen gewachsenen Weizensämlingen der Sorte "Caribo" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit 0,012- und 0,006 %-igen (Gew.%) wäßrigen Spritzbrühen, die 80 % Wirkstoff und 20 % Ligninsulfonat in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

| Wirkstoff des Beispiels | Befall der Blätter nach Spritzung mit ...%-iger Wirkstoffbrühe | |
|---|---|---|
| | 0,012 | 0,006 |
| 1 | 0 | 0 |
| 3 | 0 | 1 |
| 4 | 0 | 0 |
| 5 | 0 | 1 |
| 6 | 1 | 2 |
| 7 | 0 | 0 |
| 8 | 0 | 0 |
| 21 | 1 | 3 |
| Vergleichssubstanz | 5 | 5 |
| Kontrolle (unbehandelt) | 5 | |

0 = kein Pilzbefall, abgestuft bis 5 = Totalbefall

Patentansprüche

1. ß-Triazolylether der allgemeinen Formel I

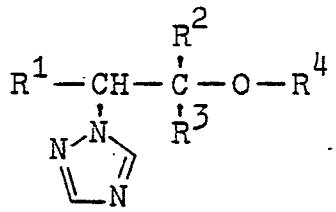

I,

in welcher

$R^1$ $C_{1-6}$-Alkyl oder gegebenenfalls halogensubstituiertes Benzyl,

$R^2$ $C_{1-6}$-Alkyl

$R^3$ Wasserstoff, $C_{1-6}$-Alkyl, $C_{2-6}$-Alkenyl, $C_{2-6}$-Alkinyl oder gegebenenfalls halogensubstituiertes Benzyl und

$R^4$ einen $C_{1-6}$-Alkyl-, $C_{3-6}$-Alkenyl-, $C_{3-6}$-Alkinyl-, oder einen gegebenenfalls halogensubstituierten Benzylrest,

bedeuten, sowie deren für Pflanzen verträgliche Salze und Metallkomplexe.

2. Verbindung ausgewählt aus der Gruppe bestehend aus

1-(2,4-Dichlorphenyl)-2-(1,2,4-triazol-1-yl)-4,4-dimethylpentyl-3-allylether

1-(4-Chlorphenyl)-2-(1,2,4-triazol-1-yl)-4,4-dimethylpentyl-3-allylether

1-(2,4-Dichlorphenyl)-2-(1,2,4-triazol-1-yl)-4,4-dimethylpentyl-3-ethylether

1-(2,4-Dichlorphenyl)-2-(1,2,4-triazol-1-yl)-4,4-dimethylpentyl-3-benzylether

1-(4-Chlorphenyl)-2-(1,2,4-triazol-1-yl)-4,4-dimethylpentyl-3-ethylether

1-(4-Chlorphenyl)-2-(1,2,4-triazol-1-yl)-4,4-dimethylpentyl-3-propin-2-yl-1-ether

1-(4-Chlorphenyl)-2-(1,2,4-triazol-1-yl)-4,4-dimethylpentyl-3-(4-chlorbenzyl)-ether

3. Verfahren zur Herstellung von ß-Triazolylethern der allgemeinen Formel I gemäß Anspruch 1, <u>dadurch gekennzeichnet</u>, daß man Alkohole der Formel II,

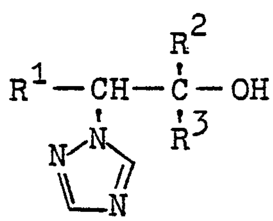

II,

worin $R^1$, $R^2$ und $R^3$ die im Anspruch 1 angegebenen Bedeutungen haben, oder deren Alkali- oder quartären Ammoniumsalze mit einem Alkylierungsmittel der Formel III

$$L - R^4 \quad III,$$

worin $R^4$ die im Anspruch 1 erläuterte Bedeutung hat und L für eine nucleophil verdrängbare Abgangsgruppe steht, gegebenenfalls in Gegenwart eines Lösungs- oder Verdünnungsmittels und/oder anorganischer oder organischer Basen und/oder eines Reaktionsbeschleunigers bei Temperaturen zwischen 0 und 100°C umsetzt,

und die so erhaltenen Verbindungen gegebenenfalls in ihre Säureadditionssalze oder Metallkomplexe überführt.

4. Fungizides Mittel, enthaltend mindestens eine Verbindung gemäß Anspruch 1.

5. Fungizides Mittel, enthaltend mindestens eine Verbindung gemäß Anspruch 1, und einen festen oder flüssigen Trägerstoff.

6. Fungizides Mittel, enthaltend eine Verbindung gemäß Anspruch 2.

7. Verfahren zur Bekämpfung von Pilzen, dadurch gekennzeichnet, daß man fungizide Mittel gemäß Anspruch 3 oder 4 auf diese einwirken läßt.

8. Verfahren zur Herstellung von Fungiziden, dadurch gekennzeichnet, daß man ß-Triazolylether der Formel I gemäß Anspruch 1 mit festen oder flüssigen Trägerstoffen mischt.

9. Verfahren zur vorbeugenden Bekämpfung von Pilzen, dadurch gekennzeichnet, daß man mindestens einen ß-Triazolylether gemäß Anspruch 1 auf durch Pilzbefall bedrohte Flächen, Pflanzen oder Saatgüter einwirken läßt.

Europäisches Patentamt

# EUROPÄISCHER RECHERCHENBERICHT

0021327

Nummer der Anmeldung

EP 80 10 3374

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 556 319 (BASF)<br>* Patentansprüche *<br>---- | 1,3-9 |

KLASSIFIKATION DER ANMELDUNG (Int. Cl.3)

C 07 D 249/08
A 01 N 43/64

RECHERCHIERTE SACHGEBIETE (Int. Cl.3)

C 07 D 249/08
A 01 N 43/64

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 26-09-1980 | CREMERS |